# EUROPEAN PATENT APPLICATION

(11) **EP 1 477 496 A1**
(43) Date of publication of application: **17.11.2004**
(21) Application number: 03734634.3
(22) Date of filing: 03.02.2003
(51) Int. Cl.: C07K 14/435, A61K 38/22, A61K 47/34, A61K 47/48, A61P 19/00

(54) **PEG-CONJUGATED PTH OR PEG-CONJUGATED PTH DERIVATE**

(30) Priority: 01.02.2002 JP 2002026194
(71) Applicant: CHUGAI SEIYAKU KABUSHIKI KAISHA, Tokyo, 115-8543 (JP)
(72) Inventor: Sekimori, Yasuo, Gotenba-shi, Shizuoka 412-8513 (JP); Nakamura, Teruo, Gotenba-shi, Shizuoka 412-8513 (JP); Shimizu, Masaru, Gotenba-shi, Shizuoka 412-8513 (JP)
(74) Representative: VOSSIUS & PARTNER
(86) International application number: PCT/JP2003/001067
(87) International publication number: WO 2003/064462

(57) **Abstract**

The object of the present invention is to provide PTH or a PTH derivative, which is modified to attain increased bioavailability without losing PTH activity and to have a reduced risk of side effects. PEG conjugation to PTH or a PTH derivative increases the bioavailability while maintaining PTH activity and also reduces side effects.

## Description

### TECHNICAL FIELD

The present invention relates to polyethylene glycol-conjugated parathyroid hormone (PEG-conjugated PTH) or PEG-conjugated PTH derivatives, PEG-conjugated PTH compositions containing the same, PTH formulations containing the same as an active ingredient, and a method for preparing the same.

### BACKGROUND ART

Parathyroid hormone (PTH) is a hormone that is primarily secreted by the parathyroid glands and regulates blood calcium levels (Kronenberg, H.M. et al., Handbook of Experimental Pharmacology, Springer-Verlag, Heidelberg, Germany, pp. 185-201, 1993). It is known as one of the important hormones involved in bone metabolism. In response to the reduction of blood calcium levels, the parathyroid glands secrete PTH. PTH stimulates calcium resorption in the kidney and induces 25 vitamin D-1α hydroxylase to convert vitamin D into its active form. PTH also enhances calcium absorption from the intestinal tract with the help of active vitamin D. In bone, PTH acts on osteoblasts to stimulate bone resorption.

Parathyroid hormone-related protein (PTHrP) is composed of 141 amino acids and has been discovered to be the main agent responsible for humoral hypercalcemia maliganncy (HHM) (Suva, L.J. et al., Science, 21: 893-896, 1987).

PTH and PTHrP are known to act on the PTH1 receptor (Juppner, H. et al., Science 254: 1024-1026, 1991). The PTH1 receptor binds to both PTH and PTHrP at the same binding level and causes not only enhancement of intracellular PKA signals, but also PLC activation through elevation of intracellular calcium levels (Abou-Samra, A. B, et al., Pro. Natl. Acad. Sci. USA, 89: 2732-2736, 1992; Bringhurst, F. R. et al., Endocrinology 132: 2090-2098, 1993).

It is well known that PTH is one of the essential systemic factors for bone remodeling. When injected intermittently in vivo, PTH produces increased bone mass in ovariectomized (OVX) rats (Hock et al., 1988; Liu et al., 1991; Ibbotson, 1992) or normal rats (Hock and Gera, 1992; Dobing, 1995). It is therefore recognized that bone formation stimulation is one of the physiological roles involved in the in vivo pulsatory secretion of PTH. In fact, both PTH(1-84) and PTH(1-34) have been clinically reported to have a more potent effect on stimulating bone formation than other drugs; the subcutaneous administration of PTH(1-34) greatly reduced fracture risk of the lumbar vertebra and femoral neck (Rittmaster, R. S. et al., J Clin Endocrinol Metab, 85: 2129-2134, 2000; Neer, R. M. et al., New Engl J Med 19: 1434-1441, 2001).

In contrast to this, bone morphometry was performed on parathyroidectomized (PTX) rats (Kitazawa et al., 1991) or normal dogs (Malluche et al., 1982) receiving continuous injection of PTH, indicating that bone formation and bone resorption were both increased simultaneously, but there was no net increase in bone mass. In view of these findings, it has been believed that continuous infusion of PTH is not suitable for treatment of osteoporosis, or rather, intermittent administration and rapid blood clearance of PTH are really critical for the treatment.

A hypercalcemic effect is known as a side effect of PTH. Although there is little increase in blood calcium levels of normal rats receiving intermittent administration of PTH by subcutaneous route (Fox, J. et al., Bone, 21: 163-169, 1997), PTH exhibits a hypercalcemic effect at a low dose when administered to chicks (Parson, J. A. et al., Endocrinology, 92: 454-462, 1973). The hypercalcemic effect can also be observed in thyroparathyroidectomized (TPTX) rats and PTX rats when administered with PTH (Horiuchi, N. et al., Am J Physiol, 244: E589-595, 1983; Ibrahim, M. M. et al., Cacif Tissue Int 34: 553-557, 1982). Likewise, clinical studies observe that a single subcutaneous administration of 20-40 µg hPTH(1-34) causes transient elevation of blood calcium levels at 4 to 6 hours after administration (Lindsay, R. et al., J Clin Endocrinol Metab, 77: 1535-1539, 1993, Neer, R. M. et al., New Engl J Med 19: 1434-1441, 2001). In view of the foregoing, PTH has a weak hypercalcemic effect in normal rodents, but it is expected to produce a strong hypercalcemic effect in clinical applications.

Another side effect of PTH is a hypotensive effect, and PTH(1-34) is reported to produce a hypotensive effect in rats and dogs (Pang, P. K. et al., Proc Natl Acad Sci, 77: 675-678, 1980). In rats, the hypotensive effect has also been examined for PTH analogs, among which PTH(1-34) is found to produce a strong hypotensive effect by subcutaneous route (Whitfield, J. F. et al., Calcif Tissue Int, 60: 302-308, 1997). Also in clinical applications, PTH(1-34) is reported to produce a hypotensive effect by intravenous route, along with transient headache after PTH administration (Igarashi, T. et al., Pharmatherapeutica, 3: 79-85, 1982). In clinical trial of PTH(1-34), when given at a dose of 40 µg, PTH(1-34) caused nausea and headache as side effects at a statistically significant level, whereas PTH(1-34) at a dose of 20 µg produced these side effects in the same frequency as observed in the placebo group. This concludes that a dose of 20 µg is clinically acceptable (Neer, R. M. et al., New Engl J Med 19: 1434-1441, 2001). In the case of anticancer agents, guidelines permit the clinical use of a dose that is one level lower than the dose limit which is unacceptable due to reasons of side effects. However, since it is usually important to ensure safe treatment for a long period of time in treating chronic diseases, there is a need to provide a sufficient gap between the dose limit which is unacceptable due to reasons of side effects and the dose clinically used. Thus, it cannot be said that the administration of PTH is sufficiently established to meet clinical demands, and hence further improvements are still encouraged.

In general, drugs have lower bioavailability (BA) when given by subcutaneous route than by intravenous route. In addition, PTH has the characteristic of being rapidly absorbed and metabolized when given by subcutaneous route (Fox, J. et al., Bone, 21: 163-169, 1997). The subcutaneous co-administration of PTH(1-34) and a protease inhibitor produces an enhanced hypercalcemic effect in chicks, suggesting that PTH receives protease digestion in the subcutaneous area (Parson, J. A. et al., Br J Pharmac, 66: 25-32, 1979). It is also reported that PTH(1-34) is metabolized primarily in the liver and kidney (Martin, K. et al., J Clin Invest, 58: 781-788, 1976; Martin K. J. et al., J Clin Invest, 60: 808-814, 1977).

WO96/35447 discloses a pharmaceutical administration form of PTH having an active ingredient release period of 2 to 6 hours, wherein a solution for chronic injection is continuously administered by subcutaneous route with the aid of a patch or iontophoresis. However, this administration form involves the following problems because it uses the subcutaneous route. Namely, a patch is convenient, but would impose a severe burden on patients because it is more likely to cause allergic reactions or irritation in response to additives or adhesives used for percutaneous absorption. On the other hand, iontophoresis requires large-scale equipment. In general, drugs have lower BA when given by subcutaneous route than by intravenous route. For percutaneous absorption, drugs are further required to pass through the skin, and hence it is expected that the percentage of PTH absorbed into the body is significantly reduced, leading to a wide range of individual differences and regional variations in this parameter even if PTH is given at a fixed dose. It would therefore be difficult to control drug efficacy and side effects. Moreover, WO96/35447 provides no teaching about hypercalcemia or blood pressure drop; the disclosed administration form appears to have no effect on reducing side effects.

In WO01/81415, an antibody Fc domain is attached to an activity-modulating domain of PTH/PTHrP to achieve sustained release of the resulting modified PTH/PTHrP. In this document, a bone resorption inhibitor is also co-administered to prevent the onset of hypercalcemia. However, the modified PTH/PTHrP, when given alone, has been found to produce a strong hypercalcemic effect even in normal mice. It is therefore expected to have a very strong hypercalcemic effect in clinical applications and appears to have no clinical utility.

Further, it is easily recognized that unlike laboratory animals, clinical patients with osteoporosis will show individual differences in their sensitivity to PTH attached to the Fc domain and in their level of bone resorption. They may also show individual differences in their sensitivity to a bone resorption inhibitor upon co-administration with PTH. As easily expected, it is very difficult to ensure safe co-administration of these two drugs at appropriate doses and at appropriate dose intervals. Moreover, the use of a chimeric protein with the Fc domain is associated with the risk of causing anaphylactic reactions due to its antigenicity as well as the risk of developing neutralizing antibodies against PTH, particularly in osteoporosis patients in need of long-term use.

On the other hand, JP 7-196925 A discloses a method and composition for imparting desired properties to a protein by modification with a water-soluble polymer. More specifically, it discloses a protein modified by covalent attachment between an aldehyde group, a ketone group, a lactol, an activated carboxylic acid or an activated carboxylic acid derivative present on the protein and a derivative of a water-soluble polymer capable of forming a hydrazone or oxime linkage (e.g., PEG) or an oxylamine water-soluble polymer. Such a modified protein attains various properties, e.g., increased solubility in an aqueous solution, increased stability during storage, reduced immunogenicity, increased resistance to enzymatic digestion, compatibility with a wider range of drug delivery systems, and increased *in vivo* half-life. According to this document, a water-soluble polymer (e.g., PEG) may be attached to various functional groups on amino acids or saccharides constituting a protein or glycoprotein molecule. The new properties mentioned above are not attained until 6 to 34 PEG molecules (MW: 2,000 to 12,000) are coupled per protein molecule.

However, with respect to the thus prepared PEG-modified protein or glycoprotein having many PEG molecules attached thereto, it is difficult to control the position and number of PEG molecules to be attached and also difficult to obtain uniform PEG-modified protein or glycoprotein molecules. Thus, there has been a problem in formulating the PEG-modified protein or glycoprotein obtained by this method into a pharmaceutical product.

Focusing on recent products commercially available abroad as PEG-modified protein pharmaceuticals, an interferon α2b formulation "PEGINTRON" (Schering-Plough) is conjugated with PEG having a molecular weight of 12,000, whereas a later-developed interferon α2a formulation "PEGASYS" (Hoffmann-La Roche) is conjugated with PEG having a higher molecular weight of 40,000 with the expectation of sustained drug efficacy. In this way, it has been believed that PEG chains of longer length are more advantageous in ensuring sustained drug efficacy in the development of PEG-modified protein pharmaceuticals.

Namely, conventional PTH formulations have a problem in controlling drug efficacy and side effects because of their low BA by subcutaneous, percutaneous or transmucosal route; there has been a need to develop a technique capable of improving or enhancing the BA and efficacy of PTH, as well as controlling side effects.

### DISCLOSURE OF THE INVENTION

As stated above, there are some reports on attempts to provide sustained-release PTH and to improve the immunogenicity and stability of PTH. However, these attempts have the problem of requiring complicated processes or causing enhanced side effects.

Thus, the inventors of the present invention have made various efforts to overcome the problems stated above. As a result, they have surprisingly found that PTH conjugated with polyethylene glycol (PEG) not only results in a sustained blood level of PTH, but also achieves extremely high BA by subcutaneous route and significantly prevents the risk of blood pressure drop, when compared to unconjugated PTH. In the present invention, it has further been found that there is no sudden increase in blood calcium levels as long as the length of PEG chains to be conjugated with PTH is selected from the range that is usually regarded as rather short to ensure enhanced drug efficacy, indicating that it is possible to control drug efficacy and side effects. These findings led to the completion of the present invention.

Thus, the present invention aims to provide PTH achieving high BA by subcutaneous route with a reduced risk of side effects.

Namely, the present invention provides a PEG-conjugated PTH or a PEG-conjugated PTH derivative.

Also, the present invention provides a PEG-conjugated PTH composition comprising the PEG-conjugated PTH or PEG-conjugated PTH derivative.

Further, the present invention provides a PTH formulation comprising the PEG-conjugated PTH or PEG-conjugated PTH derivative as an active ingredient in an amount effective to ensure drug efficacy.

Also, the present invention provides a [Cys]-PTH derivative obtainable by introduction of a cysteine residue(s) into PTH or a PTH derivative.

Further, the present invention provides a method for preparing the PEG-conjugated PTH or PEG-conjugated PTH derivative, which comprises performing a condensation or addition reaction between a PEG derivative and an amino group on PTH or a PTH derivative.

Also, the present invention provides a method for preparing the PEG-conjugated PTH or PEG-conjugated PTH derivative, which comprises conjugating PEG at a PEG conjugation site(s) introduced into PTH or a PTH derivative.

### BRIEF DESCRIPTION OF THE DRAWINGS

Figure 1(a) shows a silver-stained electropherogram of 5k and 20k PEG-PTHs analyzed by SDS-PAGE using a PhastGel Homogeneous 20. Figure 1(b) shows a silver-stained electropherogram of 2k PEG-PTH analyzed by SDS-PAGE using a PhastGel High Density.
Figure 2 shows a silver-stained electropherogram of 5k PEG-PTH analyzed by SDS-PAGE using a PhastGel Homogeneous 20.
Figure 3 is a graph showing changes in the plasma levels of PTH(1-34) and PEG-PTH(1-34) administered by intravenous route.
Figure 4 is a graph showing changes in the plasma levels of PTH(1-34) and PEG-PTH(1-34) administered by subcutaneous route.
Figure 5 is a graph showing the time course of corrected blood ionized calcium levels in normal mice administered with hPTH(1-34), 5k and 2k PEG-PTH(1-34).
Figure 6 is a graph showing the time course of blood pressure in rats, observed until 20 minutes after PTH administration.
Figure 7 is a graph showing the difference (expressed as mean ± standard error) between the average value of blood pressure before PTH administration and the value at the maximum blood pressure drop after administration.
Figure 8 contains graphs showing increases in the lumbar (a) and femoral (b) bone density of ovariectomized rats induced by administration of hPTH(1-34), [Cys³⁵]-hPTH(1-35) and 2k PEG-[Cys³⁵]-PTH(1-35).

### BEST MODE FOR CARRYING OUT THE INVENTION

The present invention is directed to a PEG-conjugated parathyroid hormone (PTH) or a PEG-conjugated PTH derivative.

The term "PTH" as used herein encompasses naturally occurring PTH, genetically-engineered recombinant PTH and chemically synthesized PTH. Examples include human PTH composed of 84 amino acid residues (human PTH(1-84)), in particular genetically-engineered recombinant human PTH(1-84). Likewise, the term "PTH derivative" is intended to mean a partial peptide of PTH as well as other peptides having biological activity similar to that of naturally occurring PTH, including those derived by substitution of some amino acids in PTH or a partial peptide thereof, those derived by deletion of some amino acids in PTH or a partial peptide thereof, and those derived by addition of one or more amino acids to PTH or a partial peptide thereof. The substitution, deletion and/or addition of some amino acids is intended to mean amino acid substitution, deletion and/or addition within a range which can be achieved by well-known techniques such as site-specific mutagenesis. Partial peptides of PTH include human PTH(1-9), human PTH(1-11), human PTH(1-14), human PTH(1-20), human PTH(1-28), human PTH(1-34), human PTH(1-35), human PTH(1-37), human PTH(1-38), human PTH(1-64), human PTH(35-84) and bovine PTH(1-34). The term "PTH(1-34)" refers to a partial peptide composed of 34 amino acids between the N-terminus and amino acid 34 of PTH or a C-terminally aminated form thereof (i.e., human PTH(1-34)-NH₂). Examples of amino acid substitution include substitution of amino acid 8 with leucine or norleucine, substitution of amino acid 18 with leucine or norleucine, and substitution of amino acid 34 with tyrosine.

It is also possible to use derivatives as found in WO 2001/23521, WO 2001/23427, WO 2000/31266, WO 2000/39278, WO 01/81415, and Naoto Shimizu, J. Biol. Chem., Vol. 276, Issue 52, 49003-49012, 2001.

The term "PTH" or "PTH derivative" also encompasses those modified to have cysteine residues, i.e., [Cys]-PTH derivatives obtained by cysteine substitution of one or more amino acids in PTH or PTH derivatives and/or obtained by addition or insertion of one or more cysteine residues into PTH or PTH derivatives. Although there is no particular limitation on the position used for cysteine introduction, for example, a cysteine residue(s) may be introduced at positions other than the N-terminus, e.g., between position 9 and the C-terminus, between position 11 and the C-terminus, between position 14 and the C-terminus, between position 20 and the C-terminus, or between position 28 and the C-terminus, and particularly at the C-terminus. By way of example, it is possible to use [Cys³⁵]-PTH(1-35) obtained by addition of a cysteine residue at the C-terminus of PTH(1-34). As used herein, introduction of a cysteine residue between position 9 and the C-terminus, given as an example, is intended to mean substitution of an amino acid residue with a cysteine residue at any position between position 9 and the C-terminus, insertion of a cysteine residue at any site, from between positions 9 and 10 to between penultimate and terminal amino acid residues in the C-terminus, as well as addition of a cysteine residue to the C-terminal amino acid residue.

The PTH or PTH derivative used in the present invention may be substantially pure and do not necessarily have 100% purity. The phrase "substantially pure" as used herein is intended to mean a product purified at least to the extent that a single peak is observed by HPLC, and particularly a product confirmed as a single band by combined electrophoretic techniques (e.g., SDS-PAGE, capillary electrophoresis, etc.). Such PTH or PTH derivative may be prepared and confirmed as described in JP 6-87897 A (corresponding to US Patent No. 5,208,041) or as described in Japanese Patent Domestic Announcement No. 4-505259 (corresponding to WO90/14415) and J. Biol. Chem., 265, 15854, 1990, with or without modifications.

In the present invention, PEG conjugation can be accomplished by reacting PTH or a PTH derivative with a PEG derivative having an active ester at one end. Examples of a PEG derivative used in the present invention include those modified to have one methoxylated end. Such PEG derivatives may further be modified to have, at the other non-methoxylated end, a functional group capable of condensation with or addition to an amino or thiol group for the purpose of conjugation with PTH or a PTH derivative. By way of example, it is possible to use succinimidyl lower fatty acid ester-PEG, aldehyde-PEG, benzotriazole carbonate-PEG, glycidyl ether-PEG, oxycarbonylimidazole-PEG, nitrophenyl carbonate-PEG and isocyanate-PEG, such as, methoxy-PEG-succinimidyl lower fatty acid esters, particularly succinimidyl propionate-methoxy PEG (mPEG-SPA) or succinimidyl butyrate-methoxy PEG (mPEG-SBA). These PEG derivatives can be conjugated to amino groups on protein molecules in the manner as described in: e.g., US Patent No. 5,672,662; WO96/11953; WO94/13222; Cristina, M. et al., Bioconjugate Chem, 6: 62-69, 1995; Charles, O. Beuchamp, et al., Anal Biochem, 131: 25-33, 1983; and Steven, M. Chamow et al., Bioconjugate Chem, 5: 133-140, 1994.

Alternatively, it is possible to use maleimide-, vinylsulfone-, ortho-pyridyl disulfide- or iodoacetamide-PEG derivatives. These derivatives may be used for addition of a cysteine residue at the terminus or within the amino acid sequence of PTH or used for conjugation with a PTH derivative modified by cysteine substitution, thus limiting the site for PEG conjugation. Addition of a cysteine residue(s) at the limited site(s) or conjugation with a protein modified by cysteine substitution may be accomplished as described in Japanese Patent Domestic Announcement No. 4-504801 (corresponding to WO90/12874), WO99/03887 and WO00/42175. The thus prepared PEG-conjugated PTH or PEG-conjugated PTH derivative according to the present invention may have 1 to 4 PEG molecules per molecule of PTH or PTH derivative. Mono-methoxy PEG-PTH (mono-mPEG-PTH) has one PEG molecule per molecule of PTH or PTH derivative.

For example, when PEG is conjugated at the C-terminus of [Cys³⁵]-PTH(1-35), such conjugation results in higher activity (e.g., enhanced *in vitro* ability to produce cAMP), more uniform quality (e.g., improved quality standard) and higher utility than random conjugation between PEG and amino groups. If the site for PEG conjugation is not controlled, PEG is frequently conjugated to the N-terminus or Lys 13, 26 or 27. The PEG-conjugated PTH or PTH derivative thus prepared (without controlling the site for conjugation) tends to show slightly lower activity in in vivo and in vitro tests (using cells not highly expressing PTH receptors), when compared to those conjugated at the C-terminus (data not shown).

The molecular weight of PEG used for modification can be changed as appropriate for the levels of sustained efficacy required by the resulting PEG-conjugated PTH or PTH derivative and side effects thereof as well as for the degree of reduction in PTH activity, etc. The molecular weight of one PEG molecule is at least 1 kDa but less than 20 kDa, preferably at least 1 kDa up to 5 kDa, more preferably at least 2 kDa up to 5 kDa, and particularly 2 kDa. PEG molecules used for conjugation may be not only linear, but also have a branched structure or a star form, etc.

In the present invention, PEG conjugation occurs at one or more sites, preferably 1 to 4 sites, in PTH or a PTH derivative. Although there is no particular limitation on the site used for conjugation, for example, PEG conjugation may occur at sites other than the N-terminus, e.g., between position 9 and the C-terminus, between position 11 and the C-terminus, between position 14 and the C-terminus, between position 20 and the C-terminus, or between position 28 and the C-terminus, and particularly at the C-terminus. Until now, it has been confirmed that PTH(1-9), PTH(1-11), PTH(1-14) and PTH(1-20) also have PTH activity (Luck, MD. et al., Mol. Endocrinol. 1999, 13, 670-680; Shimizu, M. et al., J. Bio. Chem. 2000, 275, 21836-21843; Shimizu, M. et al., Biochemistry 2002, 41, 13224-13233). It has also been known that PTH(1-27) has weaker activity than PTH(1-28) (Takasu, H. et al., Biochemistry, 38, 13453-13460, 1999). Thus, PTH is preferably conjugated with PEG at a site(s) between position 28 and the C-terminus.

PEG conjugation may also be accomplished with the help of a site(s) for PEG conjugation introduced into PTH or a PTH derivative. Examples of PEG conjugation sites include a cysteine residue.

Techniques available for purification of PEG-conjugated PTHs or PTH derivatives include PEG/dextran two-phase partition, gel filtration chromatography, ionexchange chromatography, hydrophobic interaction chromatography, reverse phase chromatography and affinity chromatography.

Example 1 illustrates the preparation of PTH conjugated with PEG molecules having a molecular weight of 2 kDa, 5 kDa and 20 kDa (i.e., 2k PEG-PTH, 5k PEG-PTH and 20k PEG-PTH, respectively). Example 2 is intended to demonstrate that these PEG-conjugated PTHs retain the ability to produce cAMP, one of the activities inherent to PTH. Example 3 is intended to demonstrate not only that the blood levels of these PEG-conjugated PTHs depend on the molecular weight of PEG, but also that these PEG-conjugated PTHs result in significantly improved BA when compared to unconjugated PTH. Moreover, as detailed in Example 4, the hypercalcemic effect which is a problematic side effect is not greatly changed in 2k and 5k PEG-PTHs as compared to unconjugated PTH, but 20k PEG-PTH has a strong hypercalcemic effect and tends to cause sustained elevation of blood calcium levels. Example 4 is therefore intended to demonstrate that PEG-conjugated PTHs having PEG molecules up to 5 kDa are safer than the Fc domain-modified PTH found in WO01/81415. It is also intended to demonstrate that 5k PEG-PTH shows a slightly different behavior than normal PTH having an increasing effect on bone mass. Further, as detailed in Example 5, 5k PEG-PTH will also be studied for the hypotensive effect, a side effect of PTH, to demonstrate that 5k PEG-PTH causes only a very small drop in blood pressure as compared to unconjugated PTH. Example 6 is intended to demonstrate that 2 kDa PEG-conjugated PTH, prepared while controlling the site for PEG conjugation, shows an increasing effect on bone density.

The present invention is also directed to a PEG-conjugated PTH composition comprising the PEG-conjugated PTH or PEG-conjugated PTH derivative stated above, as well as a PTH formulation comprising the PEG-conjugated PTH or PEG-conjugated PTH derivative as an active ingredient in an amount effective to ensure drug efficacy.

The use of the PEG-conjugated PTH or PEG-conjugated PTH derivative of the present invention (e.g., mono-methoxy PEG-PTH) not only facilitates the control of drug efficacy and side effects because of its high BA, but also prevents side effects resulting from blood pressure drop caused by PTH administration and ensures safe and effective osteoporosis treatment without the risk of causing excessive elevation of blood calcium levels. It is therefore possible to reduce or eliminate physical and ,mental pain associated with side effects (e.g., nausea and headache) experienced by patients in need of chronic PTH administration, thereby improving the quality of life (QOL) in the patients. In addition, the provision of less painful and more effective therapy can improve the patient's compliance with medication and also reduce the number of patients with bone fracture induced by osteoporosis, thus saving medical costs for elderly patients.

The PEG-conjugated PTH or PEG-conjugated PTH derivative can be given, e.g., by subcutaneous injection on a once-a-day basis or on a once- or twice-a-week basis. For example, the once-a-day dose for adults is expected to be 1 to 200 µg/person, preferably 5 to 100 µg/person, and more preferably 5 to 50 µg/person for subcutaneous injection.

The PEG-conjugated PTH or PEG-conjugated PTH derivative of the present invention can be administered to patients by various routes such as subcutaneous injection, intravenous injection, transmucosal (e.g., transpulmonary, transnasal) application and percutaneous application.

Depending on the employed route of administration, the PEG-conjugated PTH or PEG-conjugated PTH derivative of the present invention may be formulated into any dosage form such as solutions, lyophilized preparations, prefilled syringes, painless needle- or needleless-systems for subcutaneous administration, iontophoresis preparations, or sustained release preparations for intracutaneous implantation (e.g., microcapsules, polymeric micelles, polymer-based gel preparations, liposomes). The PTH formulation of the present invention is more advantageous than conventional PTH or PTH derivatives in terms of ensuring more stability.

### EXAMPLES

The present invention will be further described in the following Examples, which are not intended to limit the scope of the invention.

### Example 1

### (1) Preparation of PEG-PTHs (samples for in vitro activity test and BA test)

mPEG 2k-SPA (21.1 mg), mPEG 5k-SPA (37.0 mg) and mPEG 20k-SPA (106.1 mg) were dissolved in 2.16 mL, 1.35 mL and 1.08 mL, respectively, of a 1 mg/mL hPTH(1-34) solution (in 20 mM acetate buffer, pH 5.0) and gently shaken at room temperature for 2 hours. The resulting solutions were diluted with 1.84 mL, 0.7 mL and 3 mL, respectively, of 20 mM acetate buffer (pH 4.0) and subjected to cation-exchange chromatography. The reaction mixture of mPEG 5k-SPA and PTH was passed through a PD-10 column equilibrated with 20 mM acetate buffer (pH 4.0) to replace the solvent before being subjected to cation-exchange chromatography. Conditions for cation-exchange chromatography are shown below.

Column: Resource S, 1 mL (Amersham Pharmacia Biotech)
- Mobile phase A:: 20 mM acetate buffer (pH 4.0)
- Mobile phase B:: 20 mM acetate buffer (pH 4.0)/1 M NaCl
- Elution program:: 0 to 10 minutes (100% Mobile phase A)
25 minutes (90% Mobile phase B)
25.5 minutes (100% Mobile phase B)
- Elution speed:: 2 mL/min

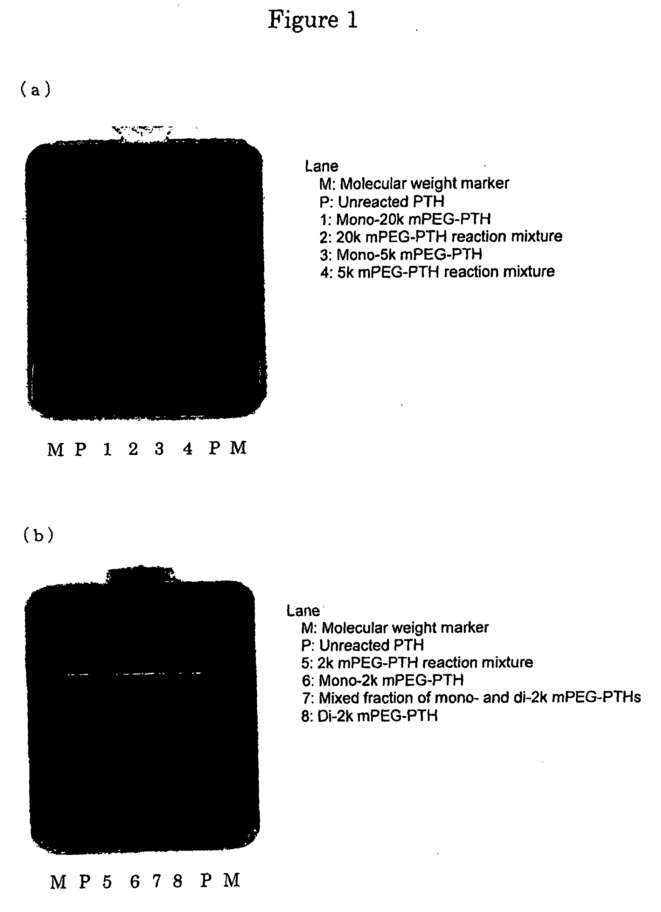

Fractions of mono-2k PEG-PTH, mono-5k PEG-PTH and mono-20k PEG-PTH, each having one PEG molecule per PTH molecule, were individually collected and concentrated with Centricon-3, 10 and 30 units to give 445 µg, 456 µg and 239 µg samples (calculated as hPTH(1-34)), respectively. Among the resulting samples, 5k PEG-PTH and 20k PEG-PTH were electrophoresed by SDS-PAGE using a PhastGel Homogeneous 20 (Figure 1a), while 2k PEG-PTH was electrophoresed by SDS-PAGE using a PhastGel High Density (Figure 1b), followed by silver staining. In the figures, Lane M is a molecular weight marker (2512, 6214, 8159, 10700, 14404, 16949 Da); Lane P, unreacted PTH; Lane 1, mono-20k PEG-PTH; Lane 2, 20k PEG-PTH reaction mixture before cation-exchange chromatography; Lane 3, mono-5k PEG-PTH; Lane 4, 5k PEG-PTH reaction mixture before cation-exchange chromatography; Lane 5, 2k PEG-PTH reaction mixture before cation-exchange chromatography; Lane 6, mono-2k PEG-PTH; Lane 7, mixed fraction of mono- and di-2k PEG-PTHs; and Lane 8, di-2k PEG-PTH.

### (2) Preparation of PEG-PTHs (samples for blood pressure drop inhibition test)

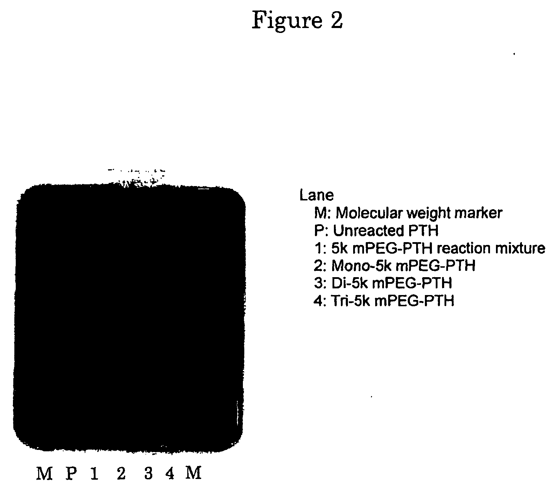

mPEG 5k-SPA (9.63 mg) was dissolved in 0.54 mL of a 1 mg/mL hPTH(1-34) solution (in 20 mM acetate buffer, pH 5.0) and gently shaken at room temperature for 2 hours. The resulting solution was diluted with 3.5 mL of 20 mM acetate buffer (pH 4.0) and subjected to cation-exchange chromatography. Conditions for cation-exchange chromatography are the same as shown in (1) above. A fraction of mono-5k PEG-PTH having one PEG molecule per PTH molecule was collected and concentrated with a Centricon-10 unit to give a 194 µg sample (calculated as hPTH(1-34)). The resulting sample was electrophoresed by SDS-PAGE using a PhastGel Homogeneous 20, followed by silver staining. Figure 2 shows the results obtained. In the figure, Lane M is a molecular weight marker; Lane P, unreacted PTH; Lane 1, 5k PEG-PTH reaction mixture before cation-exchange chromatography; Lane 2, mono-5k PEG-PTH; Lane 3, di-5k PEG-PTH; and Lane 4, tri-5k PEG-PTH.

### (3) Preparation of mPEG 2k-[Cys³⁵]-PTH(1-35)

[Cys³⁵]-PTH(1-35) (70.5 mg) was dissolved in 33.488 mL of 100 mM sodium citrate/10 mM EDTA (pH 4.0), followed by addition of 20 mM tris(2-carboxyethylphosphine) hydrochloride (1.763 mL). mPEG 2k-MAL (2 kDa methoxy-PEG-maleimide; 1.102 g) was further dissolved in the reaction mixture and allowed to stand at room temperature for 4 hours. After addition of 220 mM dithiothreitol (3.525 mL) to stop the reaction, a PD-10 column was used to replace the solvent with 20 mM sodium acetate (pH 4.0). The resulting solution was subjected in eight portions to ionexchange chromatography (under the conditions shown below) to collect a fraction of eluted mPEG 2k-[Cys³⁵]-PTH(1-35).
Column: Resource S, 6 mL
Eluent: 20 mM sodium acetate
Gradient: 0 to 1 M NaCl, 10 minutes
Detector: UV, absorbance at 280 nm

The collected fraction was concentrated and measured for its absorbance at 280 nm to determine the concentration calculated as [Cys³⁵]-PTH(1-35). The amount of the resulting mPEG 2k-[Cys³⁵]-PTH(1-35) was 48.5 mg (yield 68.8%) calculated as [Cys³⁵]-PTH(1-35).

### Example 2

### Assay for in vitro ability of PEG-PTH molecules to produce cAMP

Each PEG-PTH sample was assayed for its ability to produce cAMP (indicative of the increasing effect of PTH on bone mass) as follows. HKRK-B7 and HKRK-B64 cells, genetically modified from pig kidney-derived LLC-PK1 cells to overexpress human PTH1 receptors at levels of about 1 x 10⁶ and 9 × 10⁴ receptors per cell, respectively, as well as EW29 cells expressing about 1.9 × 10⁵ rat PTH1 receptors per cell (Takasu et al., J. Bone. Miner. Res. 14: 11-20, 1999) were grown in DMEM supplemented with 10% FBS, 1% penicillin and 1% streptomycin. These cells were seeded in 96-well plates at 1 × 10⁵ cells/well and incubated overnight. On the following day, the cells were washed once with 200 µL binding buffer (50 mM Tris-HCl/100 mM NaCl/5 mM KCl/2 mM CaCl₂/5% inactivated horse serum/0.5% inactivated bovine serum, pH 7.7), followed by addition of 100 µL cAMP assay buffer (DMEM/2 mM 3-isobutyl-1-methylxanthine/1 mg/mL bovine serum albumin/35 mM HEPES-NaOH, pH 7.4) on ice. Next, hPTH(1-34) and the PEG-PTH samples prepared in Example 1 were adjusted with the binding buffer to the respective concentrations and added in a volume of 50 µL to the cells, which were then placed in a water bath at 37°C and incubated for 20 minutes. After removing the reaction solution, the plates were placed on powdered dry ice to freeze the cells and then removed from the dry ice. The cells were thawed with 50 µL of 50 mM HCl and frozen again on dry ice. The level of intracellular cAMP was determined with a commercially available EIA kit (Biotrak CAMP EIA system, Amersham).

Table 1 shows the ability to produce cAMP in HKRK-B7 cells measured for hPTH(1-34) and each PEG-PTH(1-34) molecule. The EC₅₀ value was 1.5 nM for hPTH(1-34), while it was 0.7 nM and 1.6 nM for 2k PEG-hPTH(1-34) and 5k PEG-hPTH(1-34), respectively. This indicates that there is little effect on the ability to produce cAMP when PTH is conjugated with PEG molecules up to 5kDa. In contrast, 20k PEG-hPTH(1-34) had an EC₅₀ value of 11 nM and showed a decrease in its ability to produce cAMP, but was found to retain PTH activity.

**Table 1**

| Ability to produce cAMP measured for PEG-hPTH(1-34) | | |
|---|---|---|
| Peptide | EC₅₀ (nM) | Maximum cAMP response (pmol/well) |
| hPTH(1-34) | 1.5 | 121.3 |
| | | |
| 2k PEG-hPTH(1-34) | 0.7 | 141.2 |
| 5k PEG-hPTH(1-34) | 1.6 | 136.0 |
| 20k PEG-hPTH(1-34) | 11.0 | 122.3 |

Next, Table 2 shows the ability to produce intracellular cAMP measured for [Cys³⁵]-hPTH(1-35) and each PEG-[Cys³⁵]-PTH (1-35) molecule. In HKRK-B7 cells highly expressing human PTH1 receptors (Table 2(1)), the EC₅₀ value was 0.4 nM for hPTH(1-34), while it was 0.5 nM and 0.9 nM for 2k and 5k PEG-[Cys³⁵]-hPTH(1-35), respectively, indicating that there was little decrease in the ability to produce cAMP. In contrast, 20k PEG-[Cys³⁵]-hPTH (1-35) had an EC₅₀ value of 2.7 nM and showed a decrease in its ability to produce cAMP.

In HKRK-B64 cells with a lower expression level of human PTH1 receptors (Table 2(2)), the EC₅₀ value was 0.3 nM for hPTH(1-34), while it was 0.6 nM and 1.0 nM for 2k and 5k PEG-[Cys³⁵]-hPTH(1-35), respectively, indicating that there was little decrease in the ability to produce cAMP. In contrast, 20k PEG-[Cys³⁵]-hPTH(1-35) had an EC₅₀ value of 8.0 nM and showed a decrease in its ability to produce CAMP.

The same tendency was also observed in EW29 cells expressing rat PTH1 receptors (Table 2(3)). The EC₅₀ value was 0.2 nM for hPTH(1-34), while it was 2.2 nM and 3.5 nM for 2k and 5k PEG-[Cys³⁵]-hPTH(1-35), respectively, indicating that there was only about a 10- to 20-fold decrease in the ability to produce cAMP. In contrast, 20k PEG-[Cys³⁵]-hPTH(1-35) had an EC₅₀ value as high as 9.1 nM and showed a substantial decrease in its ability to produce cAMP.

These results indicate that conjugation with PEG molecules up to 5kDa allows the retention, to some extent, of the ability to produce cAMP, but conjugation with higher molecular weight PEG causes a significant decrease in the ability to produce cAMP.

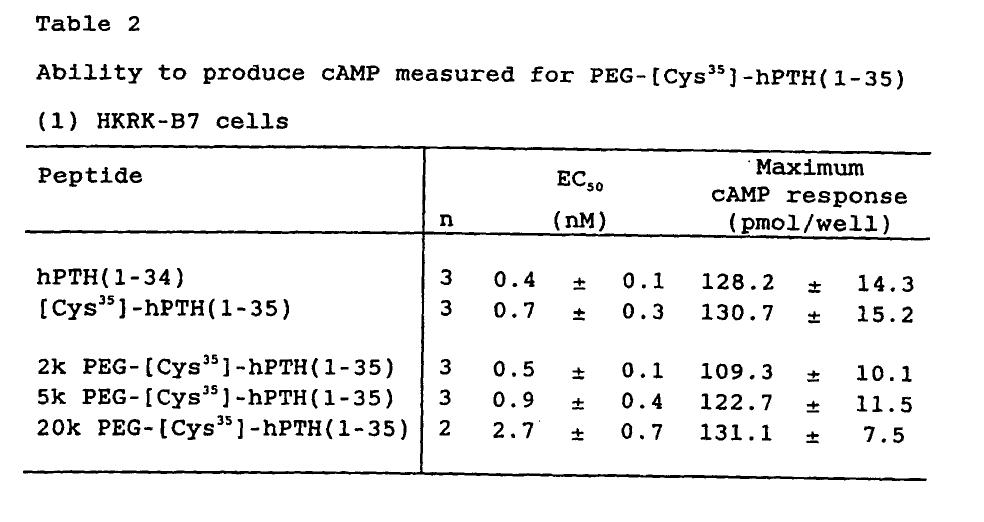

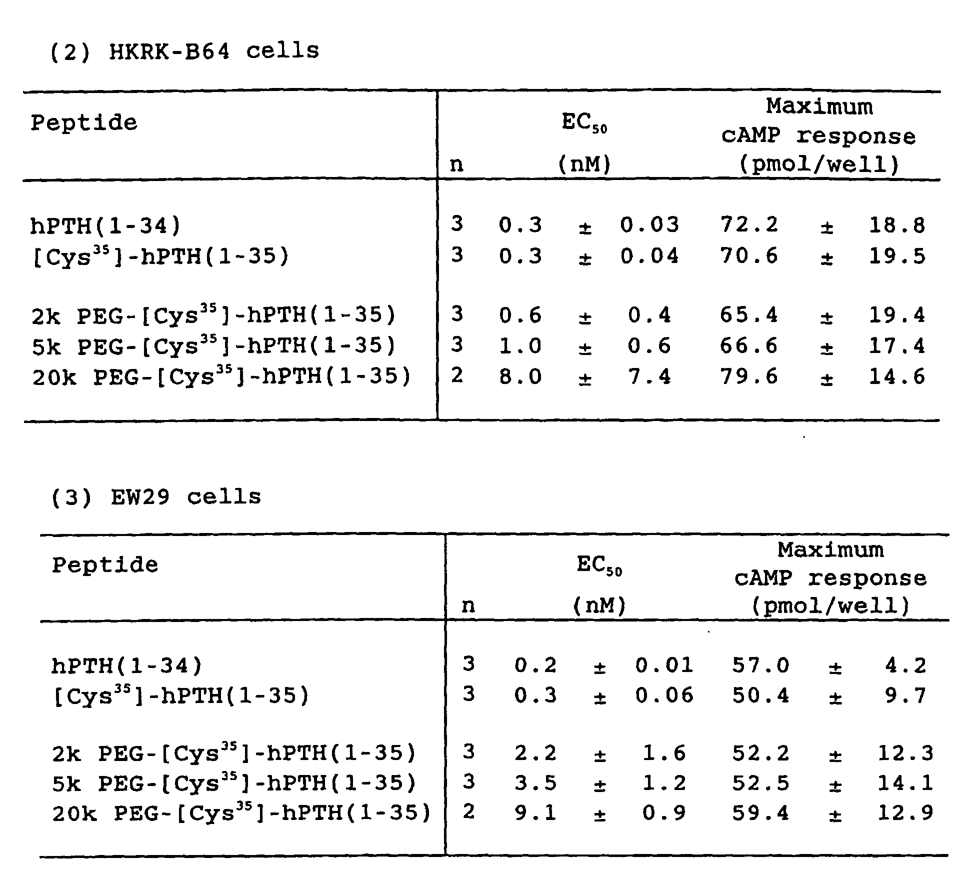

### Example 3

### Bioavailability of PEG-PTH(1-34) in rats

The mono-PEG-PTH(1-34) samples prepared in Example 1 were used to determine the bioavailability in rats.

Each sample was adjusted to 80 µg/mL by dilution with 25 mmol/L citrate-phosphate buffer (pH 5.0) containing 100 mmol/L NaCl and 0.05 vol% Tween 20 to give a solution for administration. Male SD rats at 6 weeks of age (CLEA Japan, Inc.) were provided for this experiment. Intravenous injection was performed on rats having polyethylene tubes (SP-31, Natsume Seisakusho Co., Ltd.) inserted into the femoral artery and vein. The solutions thus prepared were each administered at a dose of 1 mL/kg through a polyethylene tube inserted into the femoral vein, and blood was collected through a polyethylene tube inserted into the femoral artery before administration and at 5, 15, 30 and 45 minutes as well as 1, 2, 4, 6, 8 and 24 hours after administration. Subcutaneous administration was performed on rats having polyethylene tubes inserted into the femoral artery. The solutions prepared above were each subcutaneously administered at a dose of 1 mL/kg to the dorsal neck region of the rats, and blood was collected in the same manner as used for intravenous injection. These blood samples were centrifuged to collect plasma and stored at -40°C until assayed for PTH(1-34) or PEG-PTH(1-34) levels.

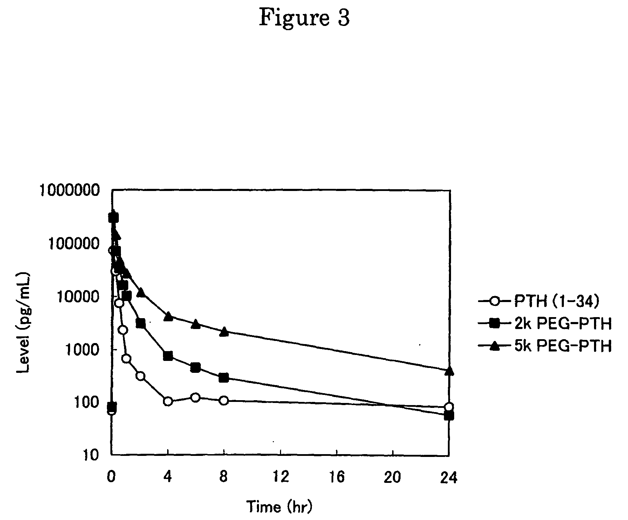

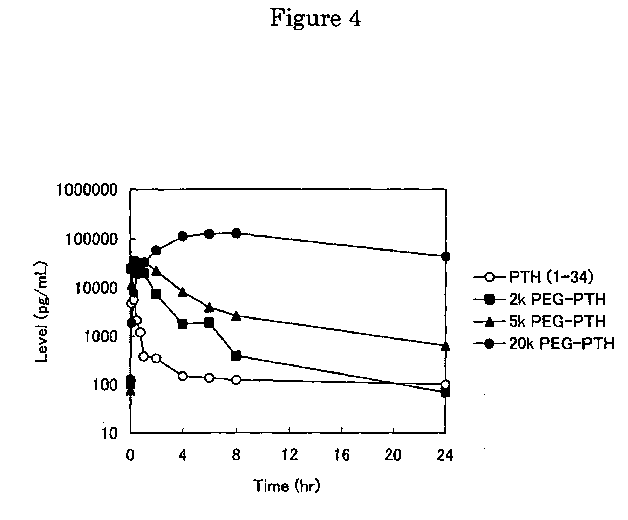

The level of PTH(1-34) or PEG-PTH(1-34) was determined by RIA analysis using a Rat PTH IRMA Kit (Immutopics). A Cobra Quantum Model 5005 gamma counter (Packard) was used to measure radioactivity for each sample. The plasma level of PTH(1-34) or PEG-PTH(1-34) was calculated from a calibration curve prepared using PTH(1-34) or PEG-PTH(1-34). The Winnonlin program Ver.2.1 (Scientific Consulting Inc.) was used to analyze the time course of changes in the calculated plasma PTH(1-34) or PEG-PTH(1-34) levels (Figure 3: by intravenous route; Figure 4: by subcutaneous route) to calculate the following as pharmacokinetic parameters: time required to reach the maximum plasma level (Tₘₐₓ); maximum plasma level (Cₘₐₓ); area under the curve of plasma levels (AUC); plasma half-life (t_{1/2}); total body clearance (Clₜₒₜₐₗ); mean residence time in the body (MRT); and bioavailability (BA). The results obtained are shown in Table 3 (by intravenous route) and in Table 4 (by subcutaneous route).

BA by subcutaneous route was about 13.6% for PTH(1-34), whereas it was increased to about 56.1% and 71.0% upon conjugation with 2kDa and 5kDa PEGs, respectively. Thus, PEG conjugation allowed PTH whose initial BA was around 10% to attain BA exceeding 50%.

This suggests that it is possible to control the time required for absorption into the blood stream, the residence capacity in the blood stream and the bioavailability by varying the molecular weight of PEG to be conjugated.

**Table 3**

| Pharmacokinetic parameters measured for PTH(1-34) and PEG-PTH(1-34) (by intravenous route) | | | | |
|---|---|---|---|---|
| | AUC (pg · h/mL) | t1/2 (h) | MRT (h) | Cltotal (mL/h/kg) |
| PTH (1-34) | 21659 | 0.231 | 0.211 | 3700.3 |
| 2k PEG-PTH (1-34) | 95781 | 1.128 | 0.465 | 841.7 |
| 5k PEG-PTH (1-34) | 178547 | 5.706 | 2.578 | 449.4 |

**Table 4**

| Pharmacokinetic parameters measured for PTH(1-34) and PEG-PTH(1-34) (by subcutaneous route) | | | | | | |
|---|---|---|---|---|---|---|
| | Tmax (h) | Cmax (h) | AUC (pg·h/mL) | t 1/2 (h) | MRT (h) | BA (%) |
| PTH (1-34) | 0.194 | 5551 | 2944 | 0.349 | 0.546 | 13.6 |
| 2k PEG-PTH (1-34) | 0.300 | 35419 | 53780 | 1.231 | 1.684 | 56.1 |
| 5k PEG-PTH (1-34) | 0.813 | 36305 | 126793 | 6.758 | 5.706 | 71.0 |
| 20k PEG-PTH (1-34) | 7.000 | 127263 | 2693945 | 11.346 | 18.93 | |

### Example 4

### Hypercalcemic effect of PEG-PTHs in normal mice

### (1) PEG-PTH(1-34)

PEG-PTH(1-34) was studied for the presence or absence of a hypercalcemic effect in normal mice as follows. hPTH(1-34) was adjusted to 100, 30 and 10 nmol/mL by dilution with 25 mmol/L phosphate-citrate buffer/100 mmol/L NaCl/0.05% Tween 80 (pH 5.0). 2k and 5k PEG-PTH(1-34) were also adjusted similarly to 30 nmol/mL (calculated as PTH) and allowed to stand on ice immediately before administration.

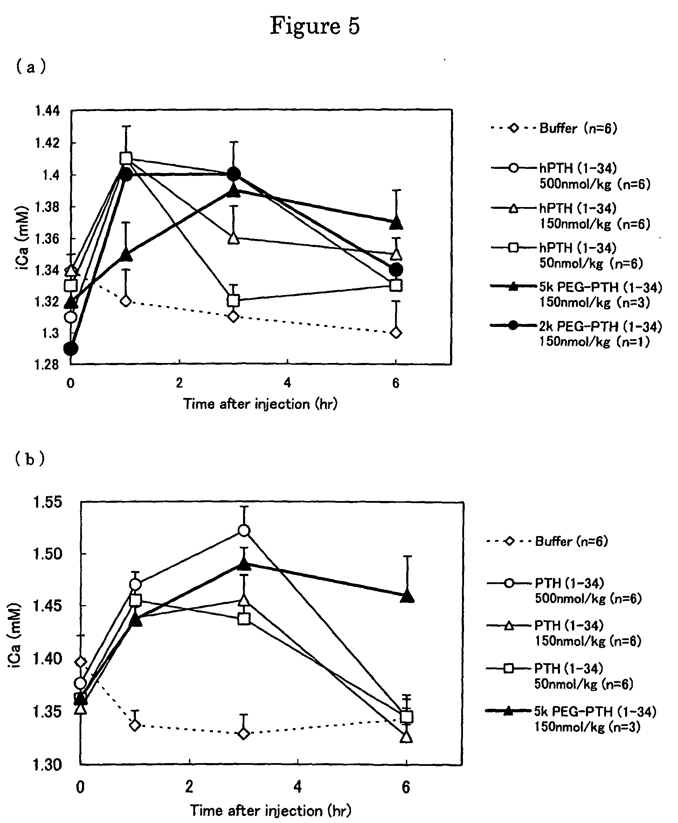

Male BDF1 mice at 5 weeks of age (Charles River Japan, Inc.) were measured for their body weight. Immediately before drug administration, blood was collected by orbital puncture into heparinized capillary tubes and measured for baseline levels of blood ionized calcium and pH using a Ca²⁺/pH analyzer (Model 348/Bayer Medical Ltd.) to give the corrected level of ionized calcium at pH 7.4 for each sample. The mice received subcutaneous administration of each drug at a dose of 5 mL/kg. The buffer as well as 500, 150 and 50 nmol/kg hPTH(1-34) were administered to groups of 6 mice each, while 150 nmol/kg 5k PEG-PTH(1-34) and 150 nmol/kg 2k PEG-PTH(1-34) were administered to three mice and one mouse, respectively. At 1, 3 and 6 hours after administration, blood was collected by orbital puncture to monitor the time course of corrected blood ionized calcium levels. Figure 5(a) shows the time course of corrected ionized calcium levels before and after administration, expressed as mean ± standard error.

As a result, all test groups had similar values for the maximum ionized calcium level and there was no great difference in this parameter when compared to differences between hPTH(1-34) and PEG-PTHs in terms of changes in their blood levels (Figure 4) and AUC (Table 4). It is therefore suggested that PEG conjugation causes no rapid increase in blood calcium levels.

When further analyzing the time course of ionized calcium levels, the ionized calcium level was gradually decreased in the buffer group, whereas each dose of hPTH(1-34) produced the maximum increase in blood ionized calcium levels at one hour after administration and then showed a decrease in this parameter. 2k PEG-PTH(1-34) also produced the maximum increase in blood ionized calcium levels at one hour after administration. The increased level was maintained until 3 hours and then decreased. When compared between 2k PEG-PTH(1-34) and hPTH(1-34), 150 nmol/kg 2k PEG-PTH(1-34) produced almost the same increase in ionized calcium levels as observed with 500 nmol/kg hPTH(1-34). In contrast, unlike hPTH(l-34) and 2k PEG-PTH(1-34), 150 nmol/kg 5k PEG-PTH(1-34) produced a gradual increase in blood ionized calcium levels to reach the same maximum value as observed with hPTH(1-34) at 3 hours after administration and then showed a tendency to maintain the increased level.

The same experiment was repeated on female DDY mice at 5 weeks of age (Charles River Japan, Inc.). The mice received subcutaneous administration of each drug at a dose of 5 mL/kg. The buffer as well as 500, 150 and 50 nmol/kg hPTH(1-34) were administered to groups of 6 mice each, while 150 nmol/kg 5k PEG-PTH(1-34) was administered to 3 mice. At 1, 3 and 6 hours after administration, blood was collected by orbital puncture to monitor the time course of corrected blood ionized calcium levels. Figure 5(b) shows the time course of corrected ionized calcium levels before and after administration, expressed as mean ± standard error.

The ionized calcium level was gradually decreased in the buffer group, whereas each dose of hPTH(1-34) produced the maximum increase in blood ionized calcium levels at 3 hours after administration and then showed a decrease in this parameter. In contrast, 150 nmol/kg 5k PEG-PTH(1-34) produced a gradual increase in blood ionized calcium levels to reach the same maximum value as observed with 500 nmol/kg hPTH(1-34) at 3 hours after administration and then showed a tendency to maintain the increased level.

### (2) PEG-[Cys³⁵]-PTH(1-35)

PEG-PTH(1-35) was studied for the presence or absence of a hypercalcemic effect in normal mice as follows. hPTH(1-34) was adjusted to 100, 30 and 10 nmol/mL by dilution with 25 mmol/L phosphate-citrate buffer/100 mmol/L NaCl/0.05% Tween 80 (pH 5.0). 2k, 5k and 20k PEG-[Cys³⁵]-PTH(1-35) were also adjusted similarly to 30, 10 and 2 nmol/mL (calculated as PTH) and allowed to stand on ice immediately before administration.

Using female DDY mice at 6 weeks of age (Japan SLC, Inc.), the corrected level of ionized calcium at pH 7.4 was determined for each sample in the same manner as shown in (1) above. The mice received subcutaneous administration of each drug at a dose of 5 mL/kg. The buffer was administered to a group of 5 mice. 500 nmol/kg hPTH(1-34) was administered to a group of 4 mice. 150 and 50 nmol/kg hPTH(1-34) were administered to groups of 5 mice each. 150 nmol/kg 2k, 5k and 20k PEG-[Cys³⁵]-PTH(1-35) were administered to groups of 4 mice each. 50 and 10 nmol/kg 2k, 5k and 20k PEG-[Cys³⁵]-PTH(1-35) were administered to groups of 5 mice each. At 1, 3, 6, 9 and 24 hours after administration, blood was collected by orbital puncture to monitor the time course of corrected blood ionized calcium levels. Table 5 shows the corrected blood ionized calcium levels before administration and until 24 hours after administration.

In the buffer group, the ionized calcium level slightly varied over time, but there was no great change. In contrast, in the hPTH(1-34) groups, this parameter increased at one hour after administration, reached the maximum value at 3 hours after administration and then decreased. The groups receiving 2k and 5k PEG-[Cys³⁵]-PTH(1-35) showed the maximum increase in blood ionized calcium levels at 3 hours after administration. The increased level was maintained until 6 hours and then decreased in each of these groups. The groups receiving 20k PEG-[Cys³⁵]-PTH (1-35) showed an increased level at 3 hours after administration and maintained a significantly higher level than that of the buffer group even at 9 hours after administration.

Further, focusing on the blood ionized calcium levels at 24 hours after administration, the groups receiving 20k PEG-[Cys³⁵]-PTH(1-35) showed very high blood ionized calcium levels at all tested doses, indicating that the mice of these groups developed hypercalcemia.

These results indicate that PTH conjugated with PEG molecules up to 5 kDa tends to slightly maintain the increasing effect on blood ionized calcium levels when compared to hPTH(1-34), although there is not so much difference in the maximum value of this effect. In contrast, conjugation with 20 kDa PEG causes hypercalcemia.

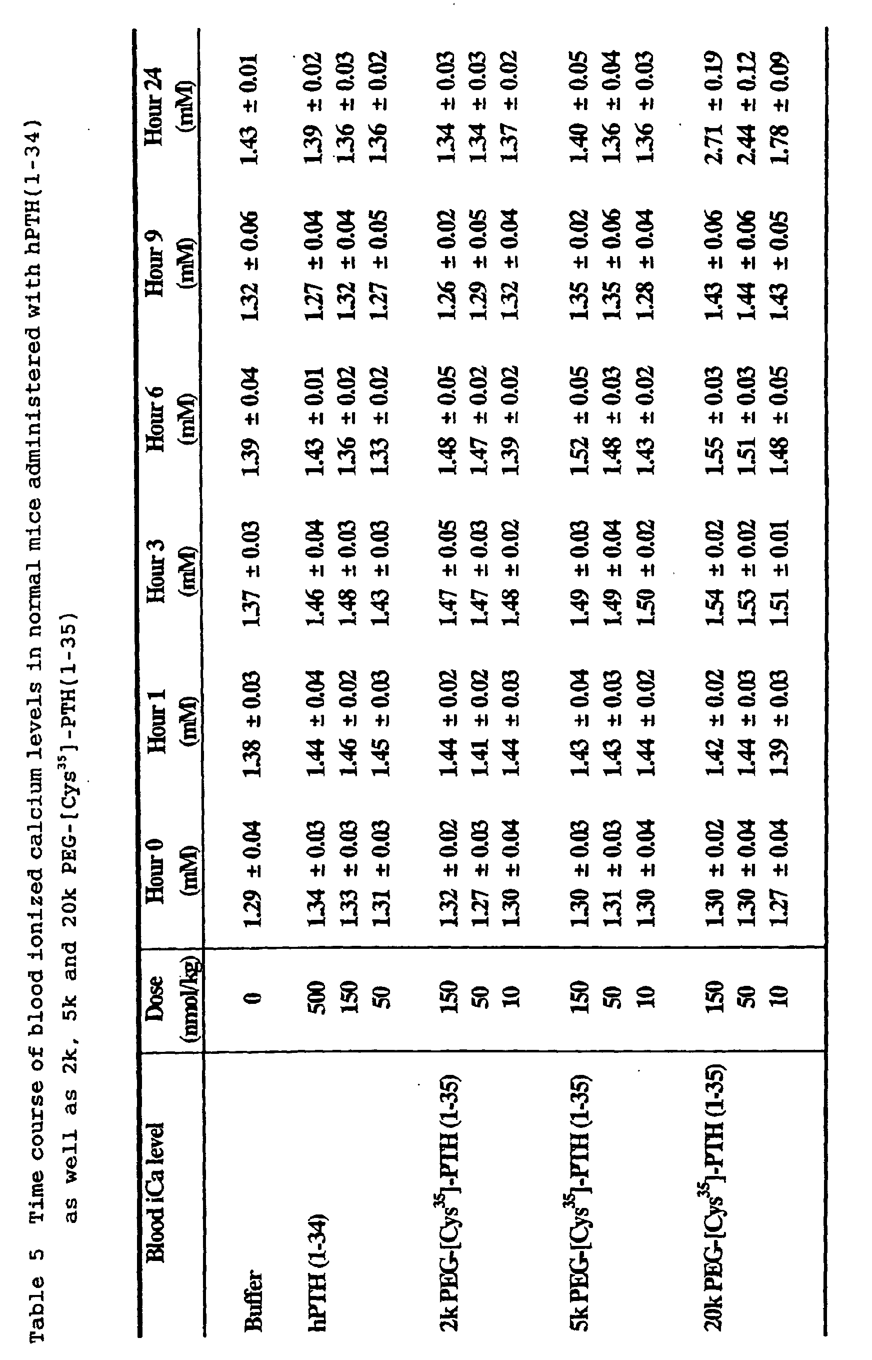

### Example 5

### Hypotensive effect of PEG-PTH(1-34) in anesthetized rats

To examine the presence or absence of side effects, PEG-PTH(1-34) was measured for its hypotensive effect in anesthetized rats as follows. hPTH(1-34) (Peptide Institute, Inc.) was adjusted to 2.5 mg/mL with 25 mM phosphate-citrate buffer, frozen and stored at -20°C until use. On the day of the test, 2.5 mg/mL hPTH(1-34) was adjusted to 50, 25 and 12.5 µg/mL by dilution with 25 mM phosphate-citrate buffer/100 mM NaCl/0.05% Tween 80 (pH 5.0). The mono-methoxy 5k PEG-PTH(1-34) prepared in Example 1(2) was also adjusted similarly to 36.5, 18.3 and 9.1 µg/mL (calculated as PTH(1-34)). The samples thus prepared were then stored on ice and restored to room temperature about 30 minutes before administration.

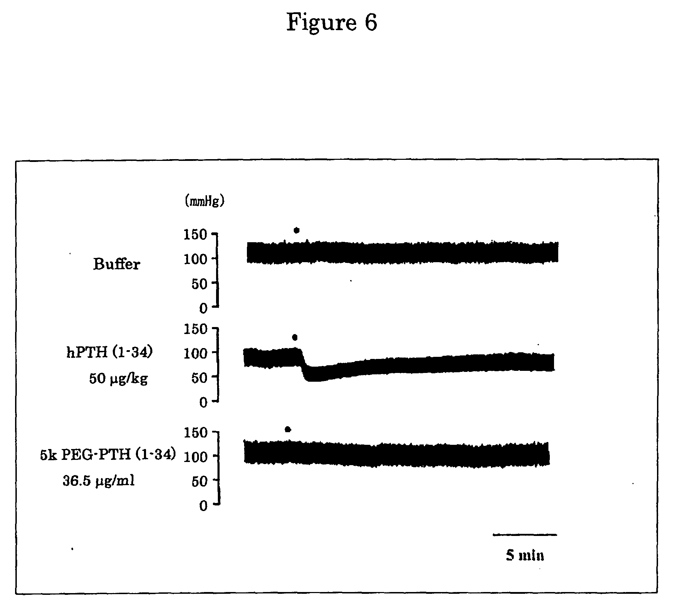

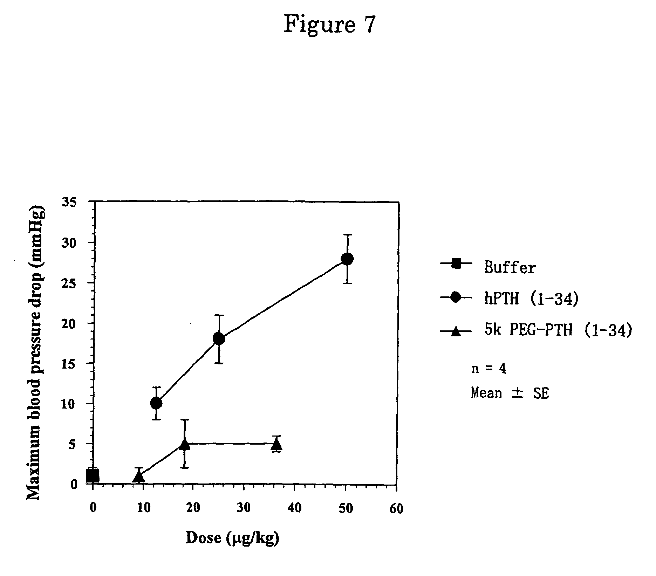

Female SD rats at 10 weeks of age (Charles River Japan, Inc.) were anesthetized by intraperitoneal administration of 50 mg/kg pentobarbital sodium (Abbott Laboratories). A catheter for blood pressure measurement and a catheter for anesthesia maintenance were inserted into the femoral artery and vein, respectively, of each rat. Anesthesia was maintained using a syringe pump (Terumo Corporation) to ensure continuous injection (at 22.5 mg/kg/hour) of pentobarbital sodium (powder form, Tokyo Kasei Kogyo Co., Ltd.) dissolved in physiological saline (Otsuka Pharmaceutical Co., Ltd.). On the other hand, each catheter for blood pressure measurement was filled with 100 U/mL heparin sodium (Novo Nordisk A/S) in physiological saline. To measure systemic blood pressure, a pressure transducer (Nihon Kohden Corporation) was connected to the catheter for blood pressure measurement and signals from the transducer were input to a blood pressure amplifier (Nihon Kohden Corporation). The resulting data were averaged for use as an indicator of systemic blood pressure. The output of systemic blood pressure was, in turn, input to a measuring unit for instantaneous heart rate (Nihon Kohden Corporation) to determine heart rate. These parameters were continuously recorded on a thermal pen recorder (Graphtec Corporation) at a speed of 5 mm/min. After surgery, an injection needle for administration of a test substance was inserted into the dorsal subcutaneous region of each rat. After each parameter had stabilized, test drugs were administered. The rats, in groups of 4, received a 1 mL/kg subcutaneous injection of either hPTH(l-34) (50, 25 and 12.5 µg/kg) or 5k PEG-PTH(1-34) (36.5, 18.3 and 9.1 µg/kg) and were monitored for blood pressure until 90 minutes after injection. Figure 6 shows a typical time course of blood pressure, observed until 20 minutes after injection. This figure illustrates changes in blood pressure induced by administration of a negative control (buffer), 50 µg/kg hPTH (1-34) and 36.5 µg/kg 5k PEG-PTH(1-34) in this order from the upper panel. In addition, the average value of blood pressure before PTH administration and the value at the maximum blood pressure drop after administration were measured for each case to determine the difference between these two values. Figure 7 shows the results expressed as mean ± standard error. The vertical axis represents the maximum blood pressure drop (mmHg) and the horizontal axis represents the amount of PTH administered. The solid square (¦), solid circle (•) and solid triangle (▲) denote buffer, hPTH(1-34) and 5k PEG-PTH(1-34), respectively.

hPTH(1-34) showed a strong hypotensive effect immediately after administration, whereas 5k PEG-PTH(1-34) had a mild hypotensive effect after administration (Figure 7). It was also indicated that hPTH(1-34) showed a dose-dependent hypotensive effect in the range of 12.5 µg/kg to 50 µg/kg, whereas 5k PEG-PTH(1-34) had a weak hypotensive effect and, in particular, produced a much weaker effect than hPTH(1-34) when administered at a higher dose (Figure 7).

### Example 6

### Increasing effect of 2k PEG-[Cys³⁵]-PTH(1-35) on bone mass in OVX rats

2k PEG-[Cys³⁵]-PTH(1-35) was studied for the increasing effect on bone mass in OVX rats as follows. By dilution with 25 mmol/L phosphate-citrate buffer/100 mmol/L NaCl/0.05% Tween 80 (pH 5.0), hPTH(1-34) and [Cys³⁵]-hPTH(1-35) were each adjusted to 10 nmol/mL, while 2k PEG-[Cys³⁵]-PTH(1-35) was adjusted to 120 nmol/ml. These samples were divided into aliquots and stored at -80°C until use.

Female SD-IGS rats at 7 weeks of age (Charles River Japan, Inc.) were used, 60 of which were ovariectomized (OVX) to remove both ovaries and 7 of which were sham-operated. After 2 weeks, the ovariectomized rats were measured for their body weight and divided into groups of 6 rats each such that the mean body weights of all groups were identical.

By dilution with the buffer, hPTH(1-34) and [Cys³⁵]-hPTH (1-35) were each adjusted to 10, 2.5 and 0.625 nmol/mL, while 2k PEG-[Cys³⁵]-PTH(1-35) was adjusted to 120, 30 and 7.5 nmol/mL.

Next, the 7 rats of the sham group received the buffer, while the ovariectomized rats, in groups of 6, were administered with the buffer, hPTH(1-34), [Cys³⁵]-hPTH (1-35) (10, 2.5 and 0.625 nmol/kg) and 2k PEG-[Cys³⁵]-PTH(1-35) (120, 30 and 7.5 nmol/kg), respectively. Each drug was given subcutaneously at a dose of 1 ml/kg on a 5 times-a-week basis for 4 weeks. On the last day of administration, the rats were housed in metabolic cages and 24-hour urine was collected from each rat. On the following day, after the rats were euthanized by exsanguination under anesthesia, an autopsy was performed to collect blood, lumbar vertebrae and femurs. The urine and blood were taken into tubes and centrifuged to collect the respective supernatants, which were stored at -20°C until assayed for parameters. The lumbar vertebrae and right femurs were stored in 70% ethanol. The average bone density of the second to fifth lumbar vertebrae and the bone density of the right femur were measured using a dual X-ray bone mineral densitometer (DCS-600EX, ALOKA).

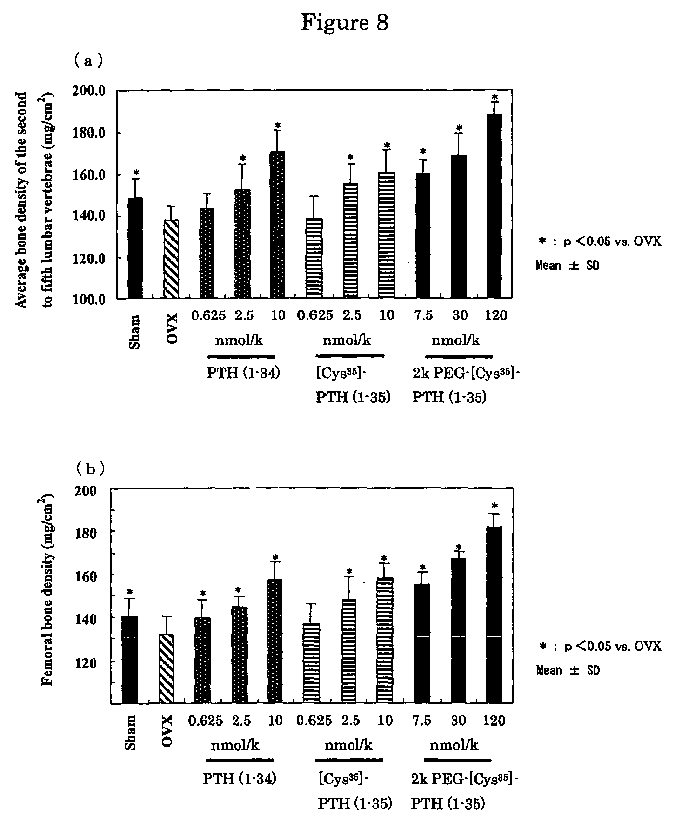

Figure 8 shows increases in the average bone density of the lumbar vertebrae (a) and the bone density of the right femur (b) induced by administration of hPTH(1-34), [Cys³⁵]-hPTH(1-35) and 2k PEG-[Cys³⁵]-PTH(1-35).

The OVX group showed statistically significant decreases in lumbar and femoral bone density over the sham group. However, administration of hPTH(1-34) and [Cys³⁵]-hPTH(1-35) caused dose-dependent increases in lumbar and femoral bone density and produced a statistically significant increasing effect on lumbar bone mass at doses of 2.5 nmol/kg and 10 nmol/kg.

In contrast, administration of 2k PEG-[Cys³⁵]-PTH(1-35) also caused dose-dependent increases in lumbar and femoral bone density and resulted in statistically significant increases in both lumbar and femoral bone density at all tested doses of 7.5 nmol/kg, 30 nmol/kg and 120 nmol/kg. Thus, 2k PEG-[Cys³⁵]-PTH (1-35) was shown to retain the increasing effect on bone mass even in vivo.

### INDUSTRIAL APPLICABILITY

When PTH or PTH derivatives are modified by PEG, such a modification allows easy provision of PEG-conjugated PTHs or PEG-conjugated PTH derivatives, which have not only increased bioavailability, but also significantly reduced side effects. Thus, the PEG-conjugated PTHs or PEG-conjugated PTH derivatives of the present invention are useful as PTH formulations for reducing the burden on patients.

## Claims

1. A polyethylene glycol (PEG)-conjugated parathyroid hormone (PTH) or a PEG-conjugated PTH derivative.

2. The PEG-conjugated PTH or PEG-conjugated PTH derivative according to claim 1, wherein PEG has a molecular weight of at least 1 kDa but less than 20 kDa.

3. The PEG-conjugated PTH or PEG-conjugated PTH derivative according to claim 2, wherein PEG has a molecular weight of at least 1 kDa up to 5 kDa.

4. The PEG-conjugated PTH or PEG-conjugated PTH derivative according to claim 3, wherein PEG has a molecular weight of at least 2 kDa up to 5 kDa.

5. The PEG-conjugated PTH or PEG-conjugated PTH derivative according to claim 4, wherein PEG has a molecular weight of 2 kDa.

6. The PEG-conjugated PTH or PEG-conjugated PTH derivative according to any one of claims 1 to 5, wherein PEG conjugation occurs at one or more sites.

7. The PEG-conjugated PTH or PEG-conjugated PTH derivative according to claim 6, wherein PEG conjugation occurs at 1 to 4 sites.

8. The PEG-conjugated PTH or PEG-conjugated PTH derivative according to claim 6 or 7, wherein PEG conjugation occurs at a site(s) other than the N-terminus.

9. The PEG-conjugated PTH or PEG-conjugated PTH derivative according to claim 8, wherein PEG conjugation occurs between position 9 and the C-terminus.

10. The PEG-conjugated PTH or PEG-conjugated PTH derivative according to claim 9, wherein PEG conjugation occurs between position 11 and the C-terminus.

11. The PEG-conjugated PTH or PEG-conjugated PTH derivative according to claim 10, wherein PEG conjugation occurs between position 14 and the C-terminus.

12. The PEG-conjugated PTH or PEG-conjugated PTH derivative according to claim 11, wherein PEG conjugation occurs between position 20 and the C-terminus.

13. The PEG-conjugated PTH or PEG-conjugated PTH derivative according to claim 12, wherein PEG conjugation occurs between position 28 and the C-terminus.

14. The PEG-conjugated PTH or PEG-conjugated PTH derivative according to claim 13, wherein PEG conjugation occurs at the C-terminus.

15. The PEG-conjugated PTH or PEG-conjugated PTH derivative according to any one of claims 6 to 14, wherein PEG is attached to PTH or a PTH derivative via a PEG conjugation site(s).

16. The PEG-conjugated PTH or PEG-conjugated PTH derivative according to claim 15, wherein each of the PEG conjugation site(s) is a cysteine residue.

17. A PEG-conjugated PTH composition, which comprises the PEG-conjugated PTH or PEG-conjugated PTH derivative according to any one of claims 1 to 16.

18. A PTH formulation, which comprises the PEG-conjugated PTH or PEG-conjugated PTH derivative according to any one of claims 1 to 16 as an active ingredient in an amount effective to ensure drug efficacy.

19. A [Cys]-PTH derivative obtainable by introduction of a cysteine residue(s) into PTH or a PTH derivative.

20. The [Cys]-PTH derivative according to claim 19, wherein the cysteine residue(s) is/are introduced at a position(s) other than the N-terminus.

21. The [Cys]-PTH derivative according to claim 20, wherein the cysteine residue(s) is/are introduced between position 9 and the C-terminus.

22. The [Cys]-PTH derivative according to claim 21, wherein the cysteine residue(s) is/are introduced between position 11 and the C-terminus.

23. The [Cys]-PTH derivative according to claim 22, wherein the cysteine residue(s) is/are introduced between position 14 and the C-terminus.

24. The [Cys]-PTH derivative according to claim 23, wherein the cysteine residue(s) is/are introduced between position 20 and the C-terminus.

25. The [Cys]-PTH derivative according to claim 24, wherein the cysteine residue(s) is/are introduced between position 28 and the C-terminus.

26. The [Cys]-PTH derivative according to claim 25, wherein the cysteine residue(s) is/are introduced at the C-terminus.

27. The [Cys]-PTH derivative according to claim 26, which is [Cys³⁵]-PTH(1-35).

28. A method for preparing the PEG-conjugated PTH or PEG-conjugated PTH derivative according to any one of claims 1 to 14, which comprises performing a condensation or addition reaction between a PEG derivative and an amino group on PTH or a PTH derivative.

29. A method for preparing the PEG-conjugated PTH or PEG-conjugated PTH derivative according to any one of claims 1 to 14, which comprises conjugating PEG at a PEG conjugation site(s) introduced into PTH or a PTH derivative.

30. The method according to claim 29, wherein each of the PEG conjugation site(s) is a cysteine residue.
